# EUROPEAN PATENT APPLICATION

(11) **EP 1 179 538 A1**
(43) Date of publication of application: **13.02.2002**
(21) Application number: 00910888.7
(22) Date of filing: 23.03.2000
(51) Int. Cl.: C07K 7/06, A61K 38/08

(54) **PEPTIDES BLOCKING RESPONSE TO CHEMICAL SUBSTANCES OR THERMAL STIMULI OR NOCICEPTOR INFLAMMATION MEDIATORS AND COMPOSITIONS CONTAINING SAID PEPTIDES**

(30) Priority: 23.03.1999 ES 9900581
(71) Applicant: Universidad Miguel Hernandez de Elche, 03806 Elche (ES)
(72) Inventor: FERRER MONTIEL, Antonio Vicente, E-03206 Elche (ES); BEIMONTE MARTINEZ, Carlos, E-03206 Elche (ES); PEREZ PAYA, Enrique, E-03206 Elche (ES); GALLAR MARTINEZ, Juana, E-03206 Elche (ES); GONZALEZ ROS, José Manuel, E-03206 Elche (ES); GALIANA GREGORI, Remedios, Maria, E-03206 Elche (ES); PLANELLS CASES, Rosa Maria, E-03206 Elche (ES)
(74) Representative: Maldonado Jordan, Julia
(86) International application number: ES0000102
(87) International publication number: WO0056761

(57) **Abstract**

Oligopeptides wherein aa1 denotes the amino acid in the N-terminus; aa2 denotes the amino acid in the C-terminus, and, X denotes an stretch of 2 to 8 identical or different amino acids, located between aa1 and aa2, and their pharmaceutically acceptable salts, which satisfy the condition that at least, 3 of the amino acids are basic or positively-charged, and at least one is an aromatic amino acid. These oligopeptides block the ionic channels that are activated by noxious stimuli such as exogenous chemical substances, thermal stimuli or inflammatory mediators, for example, the VR1 channel. These peptides are appropriate to treat or attenuate disorders mediated by the activity of this receptor, for example, pain.

aa1-X-aa2 (I)

## Description

### SCOPE OF THE INVENTION

This invention refers to peptides that are capable of blocking the response to chemical substances or thermal stimuli or nociceptor inflammation mediators, preferably by means of ionic channels inhibition, such as VR1 nocireceptor and L-glutamate ionotropic receptors, to compositions containing said peptides and to their use in the treatment of illnesses or disorders induced by the activity of said receptors, for example the sensitization of pain.

### BACKGROUND OF THE INVENTION

Pain represents a serious social and economic problem. It is calculated that more than 2 million people are daily incapacitated by suffering transitory or chronic sensations of pain. Clear examples are represented by the algesia experienced by cancer patients, migraines, arthritis, burns, accidents and surgically operated persons. In spite of the seriousness of the problem, the pharmacological arsenal to fight, prevent and/or attenuate its syndromes and progress is surprisingly limited, due, in part, to the lack of specific therapeutic dians on which to act.

The sensitization of pain initiates when the peripheral terminals of a group of sensorial neurones, known as nociceptor neurons, are activated by the harmful chemical, mechanical or thermal (1,2) stimuli (see section relative to BIBLIOGRAPHY). The nociceptor neurons transmit the information regarding tissular damage to the pain sensitization processing centres in the spinal chord and the brain.

Though the precise biological mechanisms of the pain transmission are not clearly established, the cloning and characterisation of a membrane receptor, activated by the capsaicin (from now on identified as Anocireceptor VR1≅), and which is directly involved in the pain pathways (3-5), has been recently described. The VR1 nocireceptor is specifically expressed in sensorial neurones activated by the intervention of the response to harmful chemical and thermal stimuli (3,4,6). This nocireceptor is a ligand-activated ionic channel, and is characterized in that it possesses high calcium permeability (5,7). The high VR1 nocireceptor calcium permeability is consistent with the neurodegeneration observed by the prolonged activation of nociceptor neurones, for example, under chronic pain conditions (1,5,6). In consequence, a strategy to attenuate the peripheral pain transmission and sensitization is to control the ionic channels involved in the chemical nociceptor response by means of the development of specific and potent antagonists.

In addition to the VR1 nocireceptor, the participation of the L-glutamate excitatory neurotransmitter in the transmission of pain (8-10) has also been described. The glutamate activates membrane receptors that are ionic channel activated (ionotropic receptors) or that transduce the signal through G proteins (metabotropic receptors) (11). The ionotropic glutamate receptors have been involved in glutamatergic nociception due to its Ca²⁺ ion permeability (10,11). The proposed molecular mechanism indicates that high and chronic glutamate levels cause a prolonged activation (hyperactivation) of the ionotropic receptors that charge the sensorial neurons with Ca²⁺ ions, triggering-off the massive and excessive activation of intracellular cascades that lead to the transmission of pain sensitization (2,8). In fact, it has been described that the antagonists of these receptors are capable of producing analgesia (12-17). From what has been specified, it can be deduced that a strategy to prevent or attenuate the algesia is to control the functional activity of the glutamate ionotropic receptors, specifically, those present in nociceptor neurones.

Therefore, it seems that the VR1 and the glutamate ionotropic receptors are important therapeutic dians for controlling peripheral pain in an effective manner.

In spite of the advance made in the last years, neuronal receptor inhibitors involved in the transmission of pain, that are powerful, selective and non toxic analgesics, have not yet been developed. A large part of the effort made up to now has been spent in developing opioids that recognise the opioid receptors of the central nervous system (1,2). These molecules, though analgesically powerful, present important secondary effects, such as addiction, tolerance, cognitive anomalies, etc., that limit their clinical use (17,18). An important effort has also been carried out to develop glutamate and/or glycine competitive and non competitive antagonists [a coagonist that participates in the activation of the N-methyl-D-aspartate (NDMDA) activated glutamate receptor]. These inhibitors have proved to be effective and powerful, attenuating the sensitization of pain but they have presented a restricted clinical use due, once again, to the secondary effects they present (10). The main disadvantage of using competitive and non-competitive antagonists is that these molecules interact with their receptors, inhibiting the neurotransmission unspecifically, and affecting both the glutamate pathological activity and its physiological activity (10). A strategy to overcome this therapeutic obstacle is to use non-competitive and/or competitive antagonists that are preferably joined to the agonist-receptor complex. The most important advantage in the use of these antagonists is that these agents mainly act on hyperactivated receptors (pathological receptors), showing a marginal interaction on receptors that function in rapid excitatory neurotransmission processes(physiological receptors) (10). This preferred activity on the Apathologic≅ receptors achieve that these types of antagonists be evaluated as therapeutically promising agents to prevent pain transmission (10-16). Molecules such as phencyclidine and dizolcypine are powerful competitive antagonists of the NMDA receptor that act as effective *in vitro* neuroprotectors (10, 14-16). However, its clinical use is questionable due to the psychotomimetic effects (10).

Therefore, the need to search for products capable of attenuating and/or dealing with peripheral sensitivity of pain that overcomes the previously indicated disadvantages, continues to exist.

The invention provides a solution to the existing need to understand the development of basic amino acid rich peptides capable of blocking the response to chemical substances or thermal stimuli or nociceptor inflammation mediators, preferably inhibiting the VR1 nocireceptor and the L-glutamate ionotropic receptors.

In consequence, an object of this invention is constituted by a peptide that is capable of blocking the response to chemical substances or thermal stimuli or nociceptor inflammation mediators.

An additional object of this invention is constituted by a composition that comprises said peptide, such as a pharmaceutical composition or a cosmetic composition. The use of this peptide in the elaboration of said composition constitutes another additional object of this invention.

### DETAILED DESCRIPTION OF THE INVENTION

The invention provides a general formula (I) peptide

aa1-X-aa2 (I)

where
aa1 represents the rest of the amino end amino acid;
aa2 represents the rest of the carboxyl end amino acid; and
X represents a rest of 2 to 8 equal or different amino acids, contained between aa1 and aa2,
and their pharmaceutically acceptable salts, provided that, at least 3 of the amino acids present in the sequence, are basic, and that at least 1 of the amino acids present in the sequence is an aromatic amino acid.

The formula (I) peptide, on occasions named in this description as peptide of the invention, has a length of 4 to 10 amino acids, preferably, from 5 to 8 amino acids, more preferable, 6 amino acids.

The amino acids that constitute the formula (I) peptide structural units can be natural or synthetic amino acids, and include, both the most common amino acids in proteins and the modified and less frequent amino acids in proteins.

The amino acids present in the formula (I) peptide may have configuration D or L.

Amino acids of amino (aa1) and carboxyl (aa2) ends may be the same or different.

The amino acid of the amino end (aa1) may have the acetyl end amino group, whilst the amino acid of carboxyl (aa2) end may have the amide end carboxyl group. Therefore, the present invention also includes formula (I) peptide derivatives in which the terminal amino end is acetyl and/or in which the terminal carboxyl end is amide.

Among the most common amino acids in proteins are to be found alanine, arginine, asparagine, aspartic acid, cysteine, glutamic acid, glutamine, glycine, histidine, isoleucine, leucine, lysine, methionine, phenylalanine, proline, serine, treonine, triptofane, tirosine and valine.

Among the modified amino acids, less frequent in proteins are, for example to be found, 2-aminoadipic acid, 3-aminoadipic acid, β-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, citrulline, 2,4-diaminobutyric acid, desmosine, 2,2'-diaminopimelic acid, 2,3-diaminopropyonic acid, N-ethylglycine, N-ethylasparagine, hydroxylysine, 3-hydroxyproline, 4-hydroxyproline, homociteine, homoserine, isodesmosine, N-methylglycine, N-methylisoleucine, 6-N-methyllysine, N-methylvaline, norvaline, norleucine and ornitine. This list of amino acids is illustrative, and non limitative.

In the sense it is used in this description, the term basic≅ Aamino acids refers to amino acids that contain an isoelectric point (pI) over 7, for example, arginine (pI: 10,76) and lysine (pI: 9,74), preferably, arginine. Similarly, the term Aromatic≅ Aamino acid refers to amino acids that contain at least one aromatic ring, such as phenylalanine, tyrosine and preferably, tryptophane.

The formula (I) peptide provided in this invention must contain at least 3 basic amino acids, equal or different, placed in any position of the sequence, including the amino and carboxyl ends, and may be together or separated by other amino acids. Likewise, the formula (I) peptide must contain at least 1 aromatic amino acid, which may be present in any position of the sequence. The presence of the basic amino acids, together with the aromatic amino acid seems to be necessary in order that the formula (I) peptide behaves as a potent blocker of the VR1 nocireceptor and of the glutamate ionotropic receptors since they block the ionic channel of said receptors.

The pharmaceutically acceptable salts of the formula (I) peptides provided by this invention can be found within the scope of this invention. The term Pharmaceutically acceptable≅ Asalts includes the salts normally used to form metallic salts or additive salts of acids or free bases. The nature of the salt is not critical, provided it is pharmaceutically acceptable. The pharmaceutically acceptable salts of the formula (I) peptides can be obtained as from acids or bases, organic or inorganic. Said salts may be obtained by conventional methods, well known by the experts in the art, making the appropriate acid or base react with the formula (I) peptide.

In a particular embodiment of this invention [see Example 1], the formula (I) peptide has a sequence of 6 amino acids selected from the group formed by the sequences shown in SEC. ID. N1: 1, SEC. ID. N1: 2 and SEC. ID. N1: 3 [see the section relative to the LIST OF SEQUENCES]. As can be appreciated, the peptide shown in SEC. ID. N1: 1 [Arg-Arg-Arg-Arg-Trp-Trp] has 4 consecutive rests of arginine, one of which constitutes the rest of the terminal amino end (aa1), and 2 rests of tryptophane, one of which constitutes the terminal carboxyl end (aa2). The peptide shown in SEC. ID. N1: 2 [Arg-Arg-Trp-Trp-Ile-Arg] has 3 rests of arginine, one of which constitutes the rest of the terminal amino end (aa1) and another constitutes the rest of the terminal carboxyl end (aa2), and one rest of tryptophane. Likewise, the peptide shown in SEC. ID. N1: 3 [Arg-Tyr-Tyr-Arg-Arg-Trp] has 3 rests of arginine and 3 rests of aromatic amino acids (2 Tyr and 1 Trp), one of the Arg rests constitutes the rest of the terminal amino end (aa1) whilst the rest of the Trp constitutes the rest of the terminal carboxyl end (aa2).

Additionally, the peptide of the invention may suffer reversible modifications with the purpose of enhancing its bioavailability and facilitating the passage of the hematoencephalic barrier and epitelial tissue.

The formula (I) peptide can be obtained by conventional techniques, either by means of the use of the recombining ADN technology or else by means of the chemical synthesis peptides techniques, preferably by the latter, due to the small size of the peptides. In general, said peptides are chemically synthetizied by means of conventional synthesis methods in solid phase and are purified by means of high resolution liquid chromatography (HPLC).The peptides are analysed, for example, by means of mass spectrometry.

Formula (I) peptides are capable of blocking the ionic channel activity that is characteristic of the VR1 nocireceptor expressed in *Xenopus laevis (X. laevis*) frog ovocites. It has been additionally observed, that the tested peptides (see Example 1) do not behave as competitive antagonists of the capsaicin ligand.

The capability of the formula (I) peptides to block the ionic channel of the VR1 nocireceptor expressed in *X. laevis* ovocites can be demonstrated by means of a test that evaluates the power of said peptides, blocking the agonistic-activated ionic current in *X. laevis* ovocites that express neuronal receptors, such as the VR1 nocireceptor [see Example 1.2] and glutamate ionotropic receptors (19,20). An important advantage of this biological test is that it permits the search for antagonists in functionally active receptors, enhancing the power of its use *in vivo.*

The capability of the formula (I) peptides to evaluate their analgesic effectiveness can be demonstrated by means of a test that consists in determining the effect of said peptides on the electrophysiological response of the nociceptor sensorial nervous fibres that innervate the anaesthetised cat's cornea, as is described in detail in Belmonte et al. (6). Briefly, the method consists in the dissection of the ciliary nerves that innervate the cornea, in fine nerve fillets, until a single afferent unit is located and identified by electric stimulation. The isolated fibre is subjected to electrophysiological studies that comprise the determination of the localisation and extension of the receptor field, the measure of the conduction speed and the exploration of its capsaicin Chemo-sensitization, and the heat-sensitization and machine-sensitization threshold. Once all these parameters are determined and the afferent sensorial fibre is electrophysiologically characterised, the study of its modulation by the different VR1 nocireceptor antagonists is proceeded with.

Due to the central role of the VR1 nocireceptor in the integration of pain pathways, a consequence of the blocking activity of formula (I) peptides is that of the potential development of a new family of analgesics. Moreover, the results obtained seem to indicate that formula (I) peptides, due to the blocking power of the VR1 nocireceptor they manifest, are firm candidates for the constitution of a new generation of analgesics.

The formula (I) peptides are therefore, useful as blockers of the VR1 nocireceptor ionic channel and of the glutamate ionotropic receptors *per se,* due to which, said peptides are appropriate for the treatment of illnesses and pathological alterations measured by the activity or function of the ionic channels of the VR1 nocireceptors and of the glutamate ionotropic receptors, for example, the sensitization of pain in reply to diverse harmful stimuli (mechanical, chemical and thermal).

The peptides of the invention may form part of diverse types of compositions for its application in the body of a mammal, preferably human beings. In this sense, the invention provides a composition that comprises a formula (I) peptide. In a particular embodiment, said composition is a pharmaceutical composition, whilst in the other particular embodiment, said composition is a cosmetic composition.

The pharmaceutical composition that is provided by this invention, comprises a therapeutically effective amount of a formula (I) peptide together with at least a pharmaceutically acceptable excipient

The peptides of the invention may be administered in order to treat algesias by any means that produce the contact of the peptide with the site of action of the same in the body of a mammal, preferably man.

The amount of therapeutically effective peptides that must be administered, as well as its dosage to treat a pathological condition with the peptides and/or pharmaceutical compositions of the invention shall depend on numerous factors, including age, condition of the patient, severity of the alteration or disorder, route and frequency of the administration and of the particular peptide to be used.

The pharmaceutical compositions that contain the formula (I) peptides may be presented in any form of administration, for example, solid, liquid, semi-solid, gel, ointment, cream or paste, and may be administered in any appropriate way, for example, oral, parenteral, rectum or topic, for which the pharmaceutically acceptable excipients necessary for the formulation of the desired form of administration shall be included. A revision of the different pharmaceutical forms of administration of medicaments and of the necessary excipients for the obtention of the same, may for example be found in the "ATratado de Farmacia Galénica≅" C. Faulí i Trillo,1993, Luzán 5, S.A. Ediciones, Madrid.

In consequence, an additional objective of this invention is constituted by the use of formula (I) peptides in the elaboration of a medicament for the attenuation of the nervous activity of primary sensorial neurons involved in the sensitization of pain caused by the application of hexogenous chemical substances or by thermal stimuli or by the endogenous liberation of substances by inflamed tissue, or in the elaboration of a medicament that inhibits the ionic channels activated by exogenous chemical substances or by thermal stimuli or by inflammation mediators that lead to the sensitization of pain.

More specifically, the invention refers to the use of a formula (I) peptide in the elaboration of a medicament for the treatment of pathological illnesses and disorders induced by the activity of the ionic channels of VR1 nocireceptors and of the glutamate ionotropic receptors, for example, the sensitization of pain in response to a harmful stimuli.

In a particular embodiment, the pharmaceutical composition of the invention is appropriate for the treatment of superficial burns and is presented in the form of ointment for its topic application, of similar type to those commercialised with trademarks Halibut7 or Position7.

The invention additionally provides a method for the treatment in a patient for pathological illnesses and disorders induced by the activity of the VR1 nocireceptor ionic channels and of the glutamate ionotropic receptors, for example, the sensitization of pain induced by VR1 nocireceptor and the glutamate ionotropic receptors in response to various harmful stimuli, for example, mechanical, chemical and thermal, that involves the administration to said patient suffering from said pathological illness or disorder, a therapeutically effective amount of a formula (I) peptide, preferably in the form of pharmaceutical composition containing it.

The cosmetic composition provided by this invention comprises a cosmetically effective amount of a formula (I) peptide together with at least an excipient of cosmetically acceptable adjuvant.

The peptides of the invention can be administered in cosmetic compositions in order to calm, reduce, attenuate or relieve pain or cutaneous irritation produced by thermal stimuli, for example, sun exposure, mechanical, for example, depilations, shaving, or chemical, slightly aggressive. The cosmetic composition of the invention may be presented in any appropriate form to permit the contact of the peptide with the site of action of the same on the body of the mammal on which it is applied.

The amount of peptide to be administered depends on numerous factors, among which is to be found the degree of pain or irritation produced by the thermal, mechanical or chemical stimuli, and on the peptide to be used.

The cosmetic compositions that contain the formula (I) peptide can be presented in any form of administration, for example, solid, liquid, semi-solid, gel, ointment, cream or paste, and can be administered in any appropriate way, preferably topically, for which, excipients or adjuvants shall be included, cosmetically acceptable and appropriate for the form of presentation of the cosmetic composition. In a particular embodiment, the cosmetic composition that comprises a formula (I) peptide is a product to be applied after exposure to the sun, for example, a cream, ointment of Aaftersun≅ lotion, appropriate to reduce, calm, the discomforts produced by sun burns due to the exposure to the sun. In another particular embodiment, the cosmetic composition of the invention is a product to be applied for after shaving, for example, an after shaving cream, balsam or lotion, that is appropriate for calming, reducing, attenuating or relieving cutaneous pain or irritation produced by mechanical stimuli (shaving). In another particular embodiment, the cosmetic composition of the invention is an after-depilation product, for example, an after-depilation cream, ointment or lotion, that is appropriate for calming, reducing, attenuating or relieving cutaneous pain or irritation produced by the depilation. A revision of the different forms of presentation of the cosmetic compositions and of the excipients or adjuvants necessary for their obtention can, for example, be found in "ACosmetología Teóroico-Práctica≅", Prof. A. del Pozo, published by the General Council of the Official Pharmaceutical Colleges, 30^{th} Issue, 1985.

In consequence, an additional object of this invention is constituted by the use of formula (I) peptides in the elaboration of a cosmetic composition, appropriate for calming, reducing, attenuating or relieving pain or cutaneous irritation produced by thermal, mechanical or chemical stimuli.

The invention additionally provides a cosmetic method for calming, reducing, attenuating or relieving pain or cutaneous irritation produced by thermal, mechanical or chemical stimuli in a mammal, preferably a human being, that comprises administering said mammal with an effective amount of formula (I) peptide, preferably in the form of a cosmetic composition containing it.

On the other hand, a main requirement for the identification of bioactive molecules is to perform a test that permits the determination of its biological activity on therapeutical dians. The applicant has developed a biological test that permits the evaluation of the power of molecules that block the agonist-activated ionic current in *X. laevis* ovocites expressed by neuronal receptors, such as VR1 nocireceptor and glutamatergical receptors (19,20). An important advantage of this biological test is that it permits the search for antagonists in functionally active receptors, enhancing the potentiality of its use *in vivo.*

The receptor heterological expression methods in *X. laevis* ovocites have been described in detail by Ferrer-Montiel and Montal (19).

In consequence, the invention also provides, in addition, a biological test to identify competitive antagonists of VR1 nocireceptor, appropriate as high performance screening test to screen chemotecs, combiners of diverse varied chemicals, as well as isolated molecules.

This test comprises contacting *X. laevi* ovocites expressing the VR1 nocireceptor with an agonist (ligand) for said VR1 nocireceptor, for example, capsaicin, in the presence or absence of the compound to be tested, maintaining the transmembrane voltage constant at -80 mV, and detecting the inhibiting activity of said compound to be tested by measuring the agonist-activated ionic current in the presence and absence of the compound to be tested. The power and effectiveness of the inhibiting activity of the compound to be tested can be determined by obtaining the dosage-response curves. To achieve this, the magnitude of the capsaicin-activated ionic current blockage is examined in *X. laevi* ovocites that express the VR1 nocireceptor in the presence of the growing concentrations of the compound to be tested. The ratio of ionic current intensities in the presence and absence of the compound to be tested permits the obtention of dosage-response curves (19,20). These graphs can be adjusted to logarithmic functions to determine the maximum blockage (power) and the antagonist concentration produced by half the maximum blockage (IC₅₀ effective).

The following examples serve to illustrate the nature of the present invention and shall be considered in its non limitative sense.

### EXAMPLE 1

### Peptides capable of blocking the VR1 nocireceptor

### 1.1 Peptide synthesis

The Peptides shown in SEC. ID. N1: 1, SEC. ID. N1: 2 and SEC. ID. N1: 3 have been synthesised by means of conventional synthesis methods of peptides in solid phase. The resultant peptides are purified by HPLC and then are analysed by mass spectrometry.

### 1.2 Biological activity evaluation

In order to evaluate the biological activity of peptides obtained in Example 1.1 a test was developed to evaluate the power of said peptides blocking the agonist-activated ionic current in *X. laevis* ovocites that express the VR1 nocireceptor.

The heterologous expression of receptors in *X. laevis* ovocites can be carried out according to the procedure described by Ferrer-Montiel and Montal (19). Briefly, the ovocites in X. laevis adult frogs are collected, handled and injected with cDNA that codes the VR1 nocireceptor (5, 19). The capsaicin agonist-activated ionic currents are recorded with the maintenance of the constant voltage technique with two microelectrodes (two-microelectrode voltage clamp) (19, 20). The ovocites that express the VR1 nocireceptor are transferred to the record chamber and are perfunded using an 8-exit perfusion system. The capsaicin in the absence and presence of the peptides is dissolved in a Ringer buffer (Hepes 10 mM pH 7,4 NaCl 100 mM, BaCl₂ 0,1 mM, MgCl₂ 2,0 mM, KC1 3,0 mM) supplemented with fluphenamic acid 100 µm (19,20). This buffered solution is used to minimise the contribution of the calcium-activated chloride endogenous ionic channel with the VR1 nocireceptor ionic current (19,20).The transmembrane voltage is held constant at -80 mV, and the ionic currents are activated by applying pulses of the capsaicin solution (20 µm) in the presence or absence of growing peptide concentrations. The inhibiting activity is detected by measuring the agonist -activated ionic current in the presence and absence of the peptides.

The power and effectiveness of the peptide inhibiting activity is determined by means of the obtention of dosage-response curves. To achieve this, the magnitude of the capsaicin-activated ionic current blockage is examined in ovocites that express the VR1 nocireceptor in the presence of growing peptide concentrations. The list of intensities of the ionic current in the presence and absence of the peptides is used to obtain dosage-response curves (19,20). These graphs adjust to logarithmic functions to determine the maximum blockage (power) and the antagonist concentration produced by half the maximum blockage (IC₅₀ effective).

The results obtained manifested that the 3 tested peptides (Example 1.1) blocked the ionic channel activity, characteristic of the VR1 nocireceptor expressed in *X. laevis* frog ovocites. The peptide concentrations that inhibited half of the VR1 response (IC₅₀) activated by the capsaicin to a 20 µm concentration were approximately 100 nM for the peptide identified as SEC. ID. N1: 1, approximately 2 µm for the peptide identified as SEC. ID. N1: 2, and approximately 10 µm for the peptide identified as SEC. ID. N1: 3.

It was additionally observed that these peptides did not behave as competitive antagonists of the capsaicin ligand.

Given the role of the VR1 nocireceptor in the integration of the pain pathways, a consequence of the blocking activity of these peptides is their use as analgesics. The results obtained indicate that the basic amino acid rich peptides (3 or more rests) together with, at least one aromatic amino acid, can act as powerful blockers of the VR1 nocireceptor.

### BIBLIOGRAPHY

1. Fields, H.L. (1987) Pain (McGraw-Hill), New York.
2. Baranauskas, G. and Nistri, A. (1998) Sensitization of pain pathways in the spinal cord: cellular mechanisms. ***Frog. Neurobiol.* 54**, 349-365.
3. Liu, L. and Simon, S.A. (1994) A rapid capsaicin-activated current in rat trigeminal ganglion neurons. ***Proc. Natl. Acad. Sci. USA 91,*** 738-741.
4. Szallasi, A. & Blumberg, P.M. (1996) Vanilloid receptors: new insights enhance potential as therapeutic target. ***Pain*** ***68***. 195-208.
5. Caterina, M.J., Schumacher, M.A., Tominaga, M., Rossen, T.A., Levine, J.D. and Julius, D. (1997) The capsaicin receptor: a heat-activated ion channel in the pain pathway. ***Nature 389*,** 816-824.
6. Belmonte, C., Gallar, J., Pozo, M.A. and Rebollo, I. (1991) Excitation by irritant chemical substances of sensory afferent units in the cat=s cornea. ***J. Physiol. (Lond) 437*,** 709-725.
7. García-Hirschfeld, J., López-Briones, L.G., Belmonte, C. and Valdeolmillos, M. (1994) Intracellular free calcium responses to protons and capsaicin in cultured trigeminal neurons. ***Neurosci. 67***. 235-243.
8. Sakurada, T., Wako, K., Sugiyama, A., Sakurada, C., Tan-No, K. and Kisara, K. (1998) involvement of spinal NNDA receptors in capsaicin-induced nociception.. **Pharmacol. *Biochem. Behav.* 59**. 339-345.
9. Omote, K., kawamata, T., Kawamata, M. and Namiki, A. (1998) Formalin-induced release of excitatory amino acids in the skin of rat hindpaw. ***Brain Res. 787***. 161-164.
10. Lipton, S.A. and Rosenburg.P.A. (1994). Excitatory amino acids as a final common pathway for neurologic disorders. ***New Engl. J.Med.* 330**. 613-622
11.Nakanishi S. and Masu, M. (1994) Molecular diversity and functions of glutamate receptors (1994) ***Annu. Rev.*** ***Biophys. Biomol. Struc***. ***23***, 319-348.
12.Zahn, P.K. Umali, E. and Breannan, T.J. (1998) Intrathecal non-NMDA excitatory amino acid receptor antagonists inhibit pain behaviours in a rat model of postoperative pain. ***Pain 74,*** 213-223.
13.Nicolodi, M., Del Bianco. P.L. and Sicuteri, F. (1997) Modulation of excitatory amino acids pathway: a possible therapeutic approach to chronic daily headache associated with analgesic drug abuse. ***Int. J. Clin. Pharmacol. Res.* 17,** 97-100.
14.Wiesenfeld-Hallin, Z. (1998) Combined opioid-NMDA antagonist therapies. What advantages do they offer for the control of pain syndromes? ***Drugs 55,*** *1-4*
15.Davidson, E.M. and Carlton, S.M. (1998) Intraplanar injection of dextrorphan, ketamine or memantine attenuates formalin-induced behaviours. **Brain Res. 785,** 136-142.
16.Eisenberf, E. and Pud, D. (1998) Can patients with chronic neuropathic pain be cured by acute administration of the NMDA receptor antagonist amantadine? ***Pain 74***, 337-339
17.Karlsten, R. and Gordh, T. (1997) How do drugs relieve neurogenic pain? ***Drugs Aging 11,*** 398-412.
18.González, P., Cabello, P., Germany, A., Norris, B. and Contreras, E. (1997) Decrease of tolerance to, and physical dependence on morphine by, glutamate receptor antagonists. ***Eur. J. Pharmacol.* 332**. 257-262.
19.Ferrer-Montiel, A.V. and Montal, M. (1994). Structure-function relations in ligand-gated ion channels: Reconstitution in lipid bilayers and heterologous expression in *Xenopus* oocytes. *Methods: a companion to methods in Enzymology 6,* 60-69.
20.Ferrer-Montiel, A.V., Sun, W. And Montal, M. (1995). Molecular design of the NMDA receptor binding site for PCP and MK-801. ***Proc. Natl. Acad. Sci. USA. 92*,** 8021-8025.
21.Garcia-Hirschfeld, J., López-Briones, L.G. and Belmonte, C. (1994) Neurotrophic influences on corneal epithelial cells. ***Exp. Eye Res. 59***. 597-605.
22.González, G.G., García de la Rubia, P., Gallar, J. and Belmonte, C. (1993) Reduction of capsaicin-induced ocular pain and neurogenic inflammation by calcium antagonists. ***Investig. Ophtalmol. Visual Sci. 34*,** 3329-3335.

LIST OF SEQUENCES

(1) GENERAL INFORMATION
(i) APPLICANT:
(A) NAME: Universidad Miguel Hernández
(B) ADDRESS. Centro de Biología molecular y
Celular
Universidad Miguel Hernández
Monóvar s/n
(C) CITY. Elche
(D) PROVINCE: Alicante
(E) COUNTRY: Spain
(F) POSTAL CODE: 03206
(G) TELEPHONE: 966 658 727
(H) TELEFAX. 966 658 680
(ii) TITLE OF THE INVENTION:

PEPTIDES BLOCKING RESPONSE TO CHEMICAL SUBSTANCES OR
THERMAL STIMULI OR NOCICEPTOR INFLAMMATION MEDIATORS
AND COMPOSITIONS CONTAINING SAID PEPTIDES.

(iii) NUMBER OF SEQUENCES: 3

(iv) COMPUTER READABLE FORMAT

(A) MEDIUM: DISK
(B) COMPUTER: IBM PC, compatible
(C) OPERATIVE SYSTEM. PC-DOS/MS-DOS
(D) SOFTWARE. PatentIn Release #1.0, Version
#1.30
(EPO)

(2) INFORMATION OF IDENTIFIED SEQUENCE N1 [SEC.ID.N1]: 1:

(i) CHARACTERISTICS OF THE SEQUENCE:
(A) LENGTH: 6 amino acids
(B) TYPE: AMINO ACIDS
(D) TOPOLOGY: lineal
(ii) TYPE OF MOLECULE. Peptide
(xi) DESCRIPTION OF THE SEQUENCE: SEC. ID. N1:1:

Arg Arg Arg Arg Trp Trp
1               5

(2) INFORMATION OF THE SEQUENCE IDENTIFIED N1 [SEC.ID.N1]:
2:

(i) CHARACTERISTICS OF THE SEQUENCE:
(A) LENGTH. 6 amino acids
(B) TYPE: amino acids
(D) TOPOLOGY: lineal
(ii) TYPE OF MOLECULE. Peptide
(xi) DESCRIPTION OF THE SEQUENCE. SEC. ID. N1: 2:

Arg Arg Trp Trp Ile Arg
1               5

(2) INFORMATION OF THE IDENTIFIED SEQUENCE N1[SEC.ID.N1]: 3:

(i) CHARACTERISTICS OF THE SEQLUENCE:
(A) LENGTH: 6 amino acids
(B) TYPE. Amino acids
(D) TOPOLOGY: lineal
(ii) TYPE OF MOLECULE: peptide
(xi) DESCRIPTION OF THE SEQUENCE: SEC. ID. N1: 3:

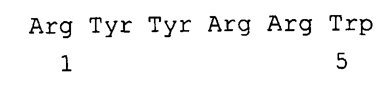

## Claims

1. A peptide of general formula (I)
aa1-X-aa2 (I)
where
aa1 represents the rest of the amino end amino acid;
aa2 represents the rest of the carboxyl end amino acid, and
X represents a rest of 2 to 8 amino acids, the same or different, contained between aa1 and aa2 and their pharmaceutically acceptable salts, provided at least 3 of the amino acids are basic, and that at least 1 of the amino acids is an aromatic amino acid.

2. Peptide according to claim 1, in which said amino acids that constitute the structural units of formula (I) peptide are selected from among natural amino acids and synthetic amino acids.

3. Peptide according to claim 1, in which said amino acids have a D configuration.

4. Peptide according to claim 1, in which said amino acids have a L configuration.

5. Peptide according to claim 1, in which said amino acids that constitute the structural units of formula (I) peptide are amino acids common in the proteins selected from among alanine, arginine, asparagine, aspartic acid, cysteine, glutamic acid, glutamine, glycine, histidine, isoleucine, leucine, lysine, methionine, phenylalanine, proline, serine, treonine, triptophane, tirosine and valine.

6. Peptide according to claim 1, in which said amino acids that constitute the structural units of the formula (I) peptide are selected from among 2-aminoadipic acid, 3-aminoadipic acid, β-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, citrulline, 2,4-diaminobutyric acid, desmosine, 2,2'-diaminopimelic acid, 2,3-diaminopropyonic acid, N-ethylglycine, N-ethylasparagine, hydroxylysine, 3-hydroxyproline, 4-hydroxyproline, homocysteine, homoserine, isodesmosine, N-methylglycine, N-methylisoleucine, 6-N-metyl-lysine, N-methylvaline, norvaline, norleucine and ornitine.

7. Peptide according to claim 1, in which the amino acids of the amino (aa1) and carboxyl (aa2) ends are the same

8. Peptide according to claim 1, in which the amino acids of the amino (aa1) and carboxyl (aa2) ends are different.

9. Peptide according to claim 1, in which the amino acid of the amino (aa1) end has the acetyl terminal amino group.

10. Peptide according to claim 1, in which the amino acid of the carboxyl (aa2) end has the amide terminal carboxyl group.

11. Peptide according to claim 1, in which said basic amino acid is selected from the group formed by amino acids that have an isoelectric point (pI) over 7.

12. Peptide according to claim 11, in which said basic amino acid is arginine.

13. Peptide according to claim 1, in which said aromatic amino acid is selected from the amino acid group that contains at least one aromatic ring.

14. Peptide according to claim 13, in which said aromatic amino acid is triptophane.

15. Peptide according to claim 1, that has an amino acid sequence selected from among the sequences of amino acids shown in SEC. ID. N1: 1, SEC. ID. N1: 2 and sec. Id. N1: 3.

16. Peptide according to claim 1, that additionally contains a reversible modification with the purpose of enhancing its bioavailability and facilitate the passage of the hematoencephalic barrier and epitelial tissue.

17. A composition that includes a formula (I) peptide according to any of claims 1 to 16.

18. A pharmaceutical composition that comprises a therapeutically effective quantity of a formula (I) peptide according to any of claims 1 to 16 and at least one pharmaceutically acceptable excipient.

19. Use of a formula (I) peptide, according to any of claims 1 to 16, in the elaboration of a medicament for nervous activity attenuation of primary sensorial neurones involved in the sensitization of pain caused by the application of exogenous chemical substances or by thermal stimuli or by the endogenous liberation of substances due to inflammed tissue.

20. Use of formula (I) peptide, according to any of claims 1 to 16, in the elaboration of a medicament that inhibits the ionic channels activated by exogenous chemical substances or by thermal stimuli or by inflammation mediators that lead to sensitization of pain.

21. Use of a formula (I) peptide according to any of claims 1 to 16, in the elaboration of a medicament for the treatment of VR1 nocireceptor ionic channels activity and of the glutamate ionotropic receptors pathological illnesses and disorders

22. Use of a peptide according to claim 20, in the elaboration of a medicament for the treatment of pain.

23. A cosmetic composition that comprises a cosmetically effective amount of a formula (I) peptide according to any of claims 1 to 16 and at least one cosmetically acceptable excipient or adjuvant.

24. Cosmetic composition according to claim 23, selected from among an after sun product, an after shave product and an after depilation product.

25. Use of a formula (I) peptide, according to any of claims 1 to 16, in the elaboration of a cosmetic composition appropriate for calming, reducing, attenuating or relieving pain or cutaneous irritation produced by thermal, mechanical or chemical stimuli.
